# EUROPEAN PATENT APPLICATION

(11) **EP 4 825 359 A1**
(43) Date of publication of application: **21.10.2026**
(21) Application number: 25171319.4
(22) Date of filing: 17.04.2025
(51) Int. Cl.: A61J 1/14, A61J 1/20, A61J 1/06, A61M 5/14

(54) **CONNECTOR DEVICE AND CONTAINER WITH A CONNECTOR DEVICE**

(71) Applicant: Röchling Medical SE, 68165 Mannheim (DE)
(72) Inventor: Scheidig, Frank, 98743 Gräfenthal (DE); Tröbs, Ralf, 07333 Unterwellenborn (DE)
(74) Representative: Ullrich & Naumann PartG mbB

(57) **Abstract**

The invention relates to an connector device for connecting a medical injection device to a container, closed by a piercable septum, the connector device comprising a proximal part for receiving the medical injection device, a distal part configured to be connected to the container, a middle part connecting the proximal part and the distal part, a first connector, preferably a hollow spike, for perforation of the piercable septum, arranged in direction from the proximal part to the distal part and a second connector, configured to reach into the medical injection device, and wherein when the medical injection device is connected to the contain-er via the connector device a fluidic connection is established between an inside of the container and an inside of the medical injection device.

## Description

The invention relates to a connector device for connecting a medical injection device to a container, closed by a piercable septum, the connector device comprising a proximal part for receiving the medical injection device, a distal part configured to be connected to the container, and a middle part connecting the proximal part and the distal part.

The invention further relates to a container, preferably a medical container, comprising an opening and a connector device for connecting the opening of the container to a medical injection device, wherein the connector device comprising a proximal part for receiving the medical injection device, and a distal part fluidly connected to the inside of the container.

Drugs, for instance dissolved in a liquid, are stored in medical containers, e.g. glass containers. To remove the drug from the container, a user uses usually a disposable plastic syringe comprising a needle to pierce a rubber septum of the medical container. However one of the drawbacks is, that the needle tip may be damaged due to the removal of the needle shield, piercing of the septum of the container and/or misalignment during insertion of the needle. Another drawback is that, during the removal process, the needle of the syringe is left free and unprotected.

To overcome these drawbacks EP 3 793 504 A2 discloses a connector for connecting a medical injection device to a container. The connector for connecting a medical injection device comprises a distal tip and a sleeve extending around the tip, the sleeve being provided with an inner threaded portion, to a container closed by a pierceable septum, said connector comprising a proximal part configured to sealingly engage the tip of the injection device, the proximal part comprising an outer threaded portion configured to be removably screwed to the inner threaded portion of the sleeve, a distal part configured to be connected to the container, and a hollow spike extending distally from the proximal part, having an internal volume configured to be in fluidic connection with the injection device, the spike being configured to perforate the pierceable septum of the container when the distal part is connected to the container, the spike comprising an opening in a distal end, said opening being configured to create a fluidic connection between the medical injection device and the container.

However one of the drawbacks is the lack of flexibility in terms of different medical injection devices. A further drawback is a low mechanical stability and therefore reliability of the fluid connection between the container and the medical injection device since the medical injection device is screwed onto the container with the needle of the medical injection device providing the fluidic connection.

An objective of the present invention is therefore to provide a connector device for connecting a medical injection device to a container which is more flexible, provides a high mechanical stability and a reliable fluid connection between the container and the medical injection device.

A further objective of the present invention is to provide an alternative connector device for connecting a medical injection device to a container.

The afore mentioned objectives may be accomplished by an embodiment of the present invention providing a connector device for connecting a medical injection device to a container, closed by a piercable septum, the connector device comprising a proximal part for receiving the medical injection device, a distal part configured to be connected to the container, a middle part connecting the proximal part and the distal part, a first connector, preferably a hollow spike, for perforation of the piercable septum, arranged in direction from the proximal part to the distal part and a second connector, configured to reach into the medical injection device, and wherein when the medical injection device is connected to the container via the connector device a fluidic connection is established between an inside of the container and an inside of the medical injection device.

The afore mentioned objectives may be accomplished by an embodiment of the present invention providing a container, preferably a medical container, comprising an opening and a connector device for connecting the opening of the container to a medical injection device, wherein the connector device comprising a proximal part for receiving the medical injection device, a distal part fluidly connected to the inside of the container, and a connecting element configured to reach into the medical injection device when the medical injection device is connected to the container via the connector device establishing a fluid connection between the inside of the container and the inside of the medical injection device.

One of the advantages that may be achieved by embodiments of the present invention is an enhanced flexibility. Not only medical injection devices with needles but also medical injection devices without any needle can be used since the septum of the container is pierced by the connector device. A further advantage that may be achieved by embodiments of the present invention is that due to the second connector a more reliable connection of the connector device with the medical injection device can be established.

Further features, advantages and further embodiments are disclosed or may became apparent in the following.

According to a preferred embodiment the second connector is hollow. This may allow for an easy and reliable fluidic connection between the container and the medical injection device. Further this enables a needle of a syringe to be more reliably attached to or guided within the connector device.

According to a further preferred embodiment the second connector is directly attached to the first connector for a direct fluidic connection between the first connector and the second connector. This may allow for a small construction space and a reliable fluidic connection.

According to a further preferred embodiment the second connector is provided in form of a spike, preferably wherein a conical end of the spike is oriented towards the medical injection device. This may allow for an at least partial insertion of the second connector into the medical injection device providing an enhanced reliability of the fluidic connection together with an enhanced mechanical stability of the connection between the connector device and the medical injection device.

According to a further preferred embodiment a cover is arranged extending from the middle part towards the distal part and beyond covering axially and circumferentially the first connector. This may provide an easy protection of the connection between the connector device and the container.

According to a further preferred embodiment the cover is adapted to releasably connect to the container, preferably by a clip-connection. This may allow for an easy installation and disassembly of the connector device.

According to a further preferred embodiment the proximal part comprises a threaded portion, preferably an outer threaded portion, for connection to a threaded portion, preferably an inner threaded portion of the medical injection device. This may allow for an easy installation and disassembly of the medical injection device with the connector device while providing a reliable connection between the connector device and the medical injection device.

According to a further preferred embodiment the proximal end comprises a projection extending axially away from the distal part, preferably wherein the threaded portion is arranged in the projection. This enhances the assembly of the connector device and the medical injection device.

According to a further preferred embodiment the projection covers axially and circumferentially the second connector. This may allow for a protection of the second connector.

According to a further preferred embodiment the second connector and the first connector have a common symmetry axis, and/or wherein an axial extension of the first connector is greater than an axial extension of the second connector. This may allow for a small construction space and a reliable fluidic connection between the container, the connector device and the medical injection device.

According to a further preferred embodiment a width of the second connector is equal to or greater than a width of the first connector. This may allow for an enhanced mechanical stability of a connection between the first and second connector and a small construction space.

According to a further preferred embodiment the first connector comprises a projection to which the second connector is attached, preferably wherein the second connector comprises a projection engaging with the projection of the first connector. This may allow for a further enhanced mechanical stability of a connection between the first and second connector and an easy attachment of the second connector to the first connector.

According to a further preferred embodiment a filter device is arranged between the proximal part and the distal part for shielding the fluidic connection from the environment. This may allow for instance to shield the fluidic connection against humidity or gases enabling a safe and sterile removal of the contents of the container.

According to a further preferred embodiment the connector device is configured to at least one of being hollow, being provided in form of a spike, preferably wherein a conical end of the spike is oriented towards the medical injection device, having a threaded portion, preferably an outer threaded portion, for connection to a threaded portion, preferably an inner threaded portion of the medical injection device, having a projection extending axially from the distal part, preferably wherein the threaded portion is arranged in the projection, having the projection covering axially and circumferentially the connecting element. This may allow for a reliable fluidic connection and/or mechanically stable connection between the container, the connector device and the medical injection device.

There are several ways how to design and further develop the teaching of the present invention in an advantageous way. To this end, it is to be referred to the patent claims subordinate to the independent patent claims on the one hand and to the following explanation of preferred examples of embodiments of the invention, illustrated by the drawing on the other hand. In connection with the explanation of the preferred embodiments of the invention by the aid of the drawing, generally preferred embodiments and further developments of the teaching will be explained.

In the drawings
- Fig. 1: shows a system of a container, a medical injection device and a connector device according to an embodiment of the present invention;
- Fig.2: shows a cross-section of the connector device according to Fig. 1; and
- Fig. 3: shows a container according to an embodiment of the present invention.

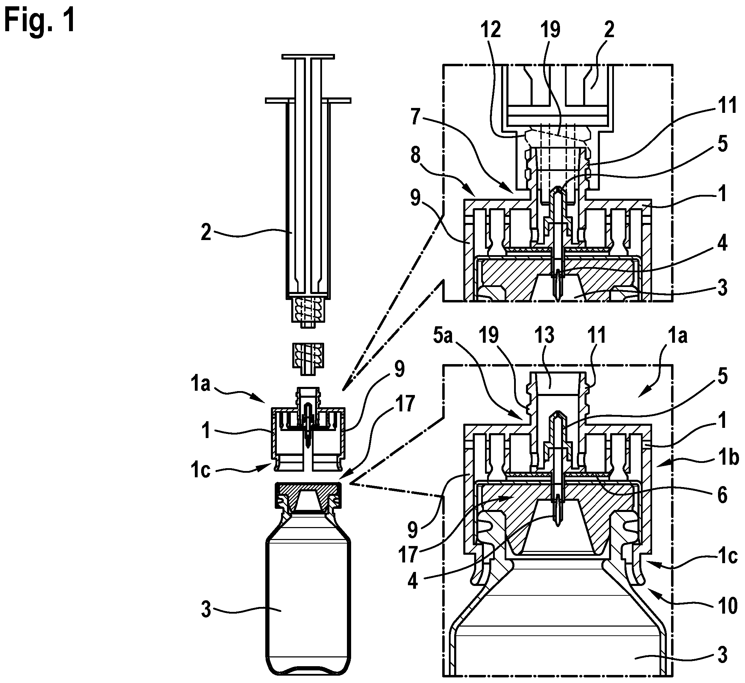

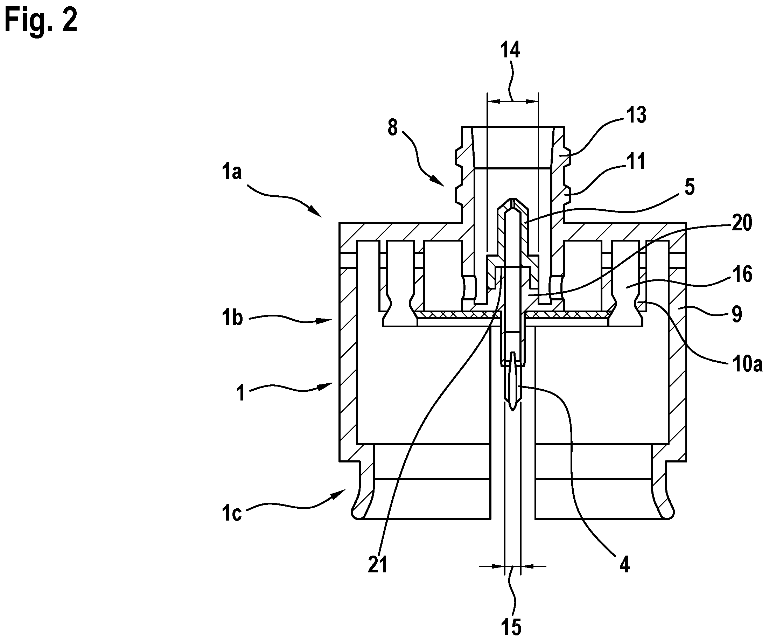

Figure 1 shows a system of a container, a medical injection device and a connector device according to an embodiment of the present invention and Fig. 2 shows a cross-section of the connector device according to Fig. 1.

In detail Figure 1 shows a container 3 with an opening 17. On this opening 17 a cylindrically formed connector device 1 can be arranged. The connector device 1 comprises a distal part 1c which is placed onto the opening 17 of the container 1. The distal part 1c of the connector device 1 further comprises a hollow spike 4 directed towards the opening 17 for piercing a piercable septum 6 of the container 1. Directly attached to the hollow spike 4 but directed towards the opposite direction and located at a proximal part 1a of the connector device 1 connected via a middle part 1b to the distal part 1c is a further hollow spike 5 the width 14 of which being larger than the width 13 of the hollow spike 4. Both hollow spikes 4, 5 provide a fluidic connection 8 between them. In Figure 1 the hollow spike 5 has the form of an upper part directed towards a medical injection device 2 and a basic part, wherein the basic part is broader than the upper part and wherein the basic part is connected to a corresponding basic part of the hollow spike 4 partially encompassing said basic part of the hollw spike 4.

The connector device 1 further comprises a cover 9 which protrudes over the opening 17 and an upper part of the container 1 ensuring via a clip-connection 10 a secure attachment of the connector device 1 to the container 3. Further a filter 16 in form of a ring within the cover 9 is provided to avoid gases or humidity to penetrate during removal of the container contents via the medical injection device 2. The filter 16 is attached via a clip connection 10a within the interior of cover 9.

At the proximal part 1a the connector device 1 comprises a projection 13 having a smaller diameter than the cover 9. The projection 13 comprises a threaded portion 11 which corresponds to a threaded portion 12 of a medical injection device 2. The medical injection device 2 comprises on the interior side of the threaded portion 12 an inner hollow projection 19 which is placed over the hollow spike 5 to provide a fluidic connection 7 between the container 3 and the medical injection device 2. When the medical injection device 2 is threaded onto the proximal end 1a of the connector device 1 the hollow spike 5 reaches in part into the medical injection device 2 with its conical end 5a. When the connector device 1 is then additionally arranged onto the opening 17 of the container 1, a fluidic connection 7 between the container 3 and the medical injection device 2 can be established.

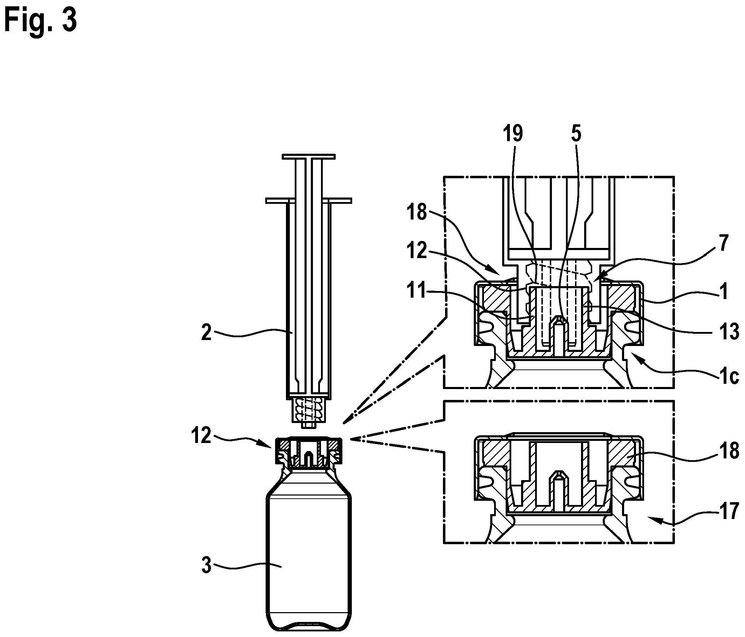

Fig. 3 shows a container according to an embodiment of the present invention.

In detail, Figure 3 shows a container 3 with integrated connector device 1 on the opening 17 of the container 3. The connector device 1 comprises a distal part providing a fluidic connection with the contents of the container 3 and a proximal part 1a for receiving the medical injection device 2. The proximal part 1a comprises a hollow spike 5 directed towards the medical injection device 2 reaching in part into the medical injection device 2 when the medical injection device 2 is arranged in fluidic connection with the proximal part 1a. The proximal part 1a comprises a projection 13 on the outer side of which a threaded portion 13 is arranged. Onto the threaded portion 11 a corresponding threaded portion 12 of the medical injection device 2 can be threaded. The medical injection device 2 comprises on the interior side of the threaded portion 12 an inner hollow projection 19 which is placed over the hollow spike 5 to provide a fluidic connection 7 between the container 3 and the medical injection device 2.

At least one embodiment may provide at least one of the following features and/or at least one of the following advantages:
- Reliable fluidic connection.
- Sterile connection.
- Easy and reliable transfer of the contents of the container to a medical injection device.
- Flexible and easy handling.
- Flexibility in terms of different medical injection devices.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### List of reference signs

- 1: connector device
- 1a: proximal part
- 1b: middle part
- 1c: distal part
- 2: medical injection device
- 3: container
- 4: hollow spike
- 5: hollow spike
- 5a: conical end
- 6: septum
- 7: fluidic connection
- 8: fluidic connection between spikes
- 9: cover
- 10, 10a: clip-connection
- 11, 12: threaded portion
- 13: projection
- 14: width second connector
- 15: width first connector
- 16: filter
- 17: opening
- 18: connecting element
- 19: inner hollow projection
- 20: projection hollow spike
- 21: projection hollow spike

## Claims

1. Connector device (1) for connecting a medical injection device (2) to a container (3), closed by a piercable septum (6), the connector device comprising
a proximal part (1a) for receiving the medical injection device (2),
a distal part (1c) configured to be connected to the container (3),
a middle part (1b) connecting the proximal part (1a) and the distal part (1c),
a first connector, preferably a hollow spike (4), for perforation of the piercable septum (6), arranged in direction from the proximal part (1a) to the distal part (1c) and
a second connector (5), configured to reach into the medical injection device (2), and wherein when the medical injection device (2) is connected to the container (3) via the connector device (1) a fluidic connection (7) is established between an inside of the container (3) and an inside of the medical injection device (2).

2. Connector device (1) according to claim 1, wherein the second connector (5) is hollow.

3. Connector device (1) according to one of the claims 1-2, wherein the second connector (5) is directly attached to the first connector (4) for a direct fluidic connection (8) between the first connector (4) and the second connector (5).

4. Connector device (1) according to one of the claims 1-3, wherein the second connector (5) is provided in form of a spike, preferably wherein a conical end (5a) of the spike (5) is oriented towards the medical injection device (2).

5. Connector device (1) according to one of the claims 1-4, wherein a cover (9) is arranged extending from the middle part (1b) towards the distal part (1c) and beyond covering axially and circumferentially the first connector (4).

6. Connector device (1) according to one of the claims 1-5, wherein the cover (9) is adapted to releasably connect to the container (3), preferably by a clip-connection (10).

7. Connector device (1) according to one of the claims 1-6, wherein the proximal part (1a) comprises a threaded portion (11), preferably an outer threaded portion (11), for connection to a threaded portion (12), preferably an inner threaded portion (12) of the medical injection device (2).

8. Connector device (1) according to one of the claims 1-7, wherein the proximal end (1a) comprises a projection (13) extending axially away from the distal part (1c), preferably wherein the threaded portion (11) is arranged in the projection (13).

9. Connector device (1) according to claim 7, wherein the projection (13) covers (9) axially and circumferentially the second connector (5).

10. Connector device (1) according to one of the claims 1-9, wherein the second connector (5) and the first connector (4) have a common symmetry axis, and/or wherein an axial extension of the first connector (4) is greater than an axial extension of the second connector (5).

11. Connector device (1) according one of the claims 1-10, wherein a width (14) of the second connector (5) is equal or greater than a width (13) of the first connector (4).

12. Connector device (1) according to one of the claims 1-11, wherein the first connector (4) comprises a projection (20) to which the second connector (5) is attached, preferably wherein the second connector (5) comprises a projection (21) engaging with the projection (20) of the first connector (4).

13. Connector device (1) according to one of the claims 1-12, wherein a filter device (16) is arranged between the proximal part (1a) and the distal part (1c) for shielding the fluidic connection (7) from the environment.

14. Container (3), preferably a medical container (3), comprising an opening (17) and a connector device (1) for connecting the opening (17) of the container (3) to a medical injection device (2), wherein the connector device (1) comprising
a proximal part (1a) for receiving the medical injection device (2),
a distal part (1c) fluidly connected to the inside of the container (3),
and a connecting element (18) configured to reach into the medical injection device (2) when the medical injection device (2) is connected to the container (3) via the connector device (1) establishing a fluidic connection (7) between the inside of the container (3) and the inside of the medical injection device (2).

15. Container (3) according to claim 14, wherein the connector device (1) is configured to at least one of
- being hollow,
- being provided in form of a spike (5), preferably wherein a conical end (5a) of the spike (5) is oriented towards the medical injection device (2),
- having a threaded portion (11), preferably an outer threaded portion (11), for connection to a threaded portion (12), preferably an inner threaded portion (12) of the medical injection device (2),
- having a projection (13) extending axially from the distal part (1c), preferably wherein the threaded portion (11) is arranged in the projection (13),
- having the projection (13) covering axially and circumferentially the connecting element (18).
